# EUROPEAN PATENT APPLICATION

(11) **EP 2 158 906 A1**
(43) Date of publication of application: **03.03.2010**
(21) Application number: 08015231.7
(22) Date of filing: 28.08.2008
(51) Int. Cl.: A61K 31/05, A61K 31/23, A61K 36/889, A61K 8/00, A61P 17/14

(54) **Composition for the treatment of alopecia**

(71) Applicant: ProxiPHARM GmbH, 8051 Zürich (CH)
(72) Inventor: Krasniqi, Nazmi, CH 8153 Rümlang (CH); Pelloni, Claudio D., 5408 Ennetbaden (CH)
(74) Representative: Dey, Michael

(57) **Abstract**

The present invention relates to the use of a combination comprising alkylesters of fatty acids, of an extract from the fruits of *Seronea repens* and of natural phenolic compounds for the preparation of a composition for the treatment of symptoms of alopecia, preferably alopecia androgenetica.

## Description

Alopecia androgenetica is frequently treated with the drugs finasterid or minoxidil. Both drugs are not ideal in respect of unwanted effects. The orally applied finasterid has negative effects on male potency due to its systemic action. The topically applied minoxidil is in fact able to block genetically determined hair loss, but hair falls out again within a short period of time after discontinuation of treatment.

Therefore, there is a need for a composition without unwanted effects which blocks not only the symptoms of alopecia androgenetica, but reactivates also cells of the roots of hairs. Surprisingly, the inventors found out that a combination of natural mixtures shows an excellent ability to reactivate the cells of the roots of human hair. Although the single components of the selected combination are already known to act against alopecia androgenetica, the new combination presented in this invention was surprisingly significantly more effective than could be expected from the additive effect of its single components.

Thus, the present invention relates to the use of a combination comprising alkylesters of fatty acids, of an extract from the fruits of *Seronea repens* and of natural phenolic compounds for the preparation of a composition for the treatment of symptoms of alopecia, preferably alopecia androgenetica. The composition may comprise further diluents, excipients, carriers, etc. which are known to the person skilled in the art.

In an especially preferred embodiment of the present invention, the alkylesters are esterified with fatty acids of chain lengths between C 7 and C 30 , containing between 0 and 2 double bonds and between 1 and 3 hydroxy- or ester groups besides the normal ester group. Particularly preferably, the alkylesters of fatty acids according to the invention are methyl-, ethyl-, propyl-, isopropyl- or butylesters.

In another preferred embodiment, the extract from *Seronea repens* contains the lipophilic constituents of the fruits.

Preferably, the natural phenolic compounds of the combination according to the present invention are selected from natural mixtures containing phenolic acids and flavonoids with phenolic substituents, preferably procyanidins.

In another preferred embodiment, but not restricted thereto, the phenolic compounds are constituents of a plant extract or mixtures of plant extracts, preferably pine bark extract (preferably made from Pinus pinaster) and/or grape seed extract.

In an especially preferred embodiment of the present invention, the combination used is characterized by a content of about 1-50% of alkylesters of fatty acids , of about 0.05-20% extract of the fruits from *Seronea repens* and of about 0.01-10% of natural phenolic compounds.

According to another preferred embodiment, the combination is characterized by addition of about 0.1-10% nettle root extracts.

Another aspect of the present invention relates to a composition as defmed above comprising alkylesters of fatty acids, an extract from the fruits of Seronea repens and natural phenolic compounds.

### The Components of the Combination

Alkylesters of fatty acids are used as cosmetics against symptoms of alopecia androgenetica. Fatty acids from avocado- or soy oil, esterified with aliphatic alcohols of a chain length between 1 and 6 carbon atoms are disclosed by WO 01/52 837 A2 for treatment of alopecia. Another possibility to treat alopecia androgenetica with natural substances offers the use of an extract of the fruits of *Seronea repens.* The extract inhibits genetic hair loss following its topical application (Prager et al., J. Altern. Complement. Med. Dic. 8, 143-52, 2002). Furthermore, proanthocyanidins or procyanidin-containing plant extracts have been used against hair loss and to stimulate hair growth.

EP 0768 079 B1 discloses the external application of a drug containing procyanidins with a short chain length for treatment of alopecia heriditaria and for hair growth. EP 1 232 740 A2 describes the topical application of procyanidins in combination with phosphatidic acids and dditional components as a hair growing agent. EP 0 573 141 B1 discloses the use of a pine bark extract (containing procyanidins) in combination with a bamboo extract and with an extract from Japanese apricots for regeneration of hair growth. All the compositions cited above seem to have no unwanted effects, however, the efficacy of these agents leaves room for improvement.

### Development of an effective combination against symptoms of alopecia androgenetica.

A basic phenomenon in the development of hair loss is the reduced rate of cell divisions of hair cells in the hair roots. To inhibit hair loss, it is of fundamental importance to stimulate the cell division of hair cells, leading to a regeneration of hair growth, thereby stopping hair loss. To screen the regenerative power of selected compositions on human hair cells the following test system was established:

Anagene hair follicles were obtained by microdissection from human volunteers aged between 24 and 50 years. The 2 mm long hair follicles were incubated in standard cell culture medium under sterile conditions at 37°C. 3 Hair follicles were placed in each well of a 24 wells cell culture plate. To the medium were added various test substances either as single components or as mixtures of the test substances. The medium in the wells, containing the test solutions, was replaced every day by fresh test solutions. Hair growth was measured after 3 days of culture under the microscope using an ocular micrometer.

The following substances were tested, Tab.1:

**Tab. 1: Hair growth after 3 days is given as difference to control in micrometers.**

| | | | |
|---|---|---|---|
| 1.Medium without test substances (control) | - | 5.Pine bark extract B | 0.10 |
| 2.Extract from fruits of Seronea repens A | 0.21 | 7.Extract from nettle herb | -0.02 |
| 3.Extract from fruits of Seronea repens B | 0.12 | 8.Extract from grape seeds | 0.42 |
| 4.Pine bark extract A | 0.26 | 9.Fatty acid alkylesters | 0.37 |

### Example 1:

The following combinations consisting of substances from Tab.1 were tested under the same conditions as for the single components, using the same concentrations as in experiments listed in Tab.1. Results (found ) are given as difference to control. The calculated value (calc) represents the added differences to control of the components contained in the mixture.

**Tab 2: Increase in hair growth (µm) relative to control in mixtures of two components (found) and calculated as additive effect of the components (calc.)**

| **Components** | **Hair growth** | | **Synergistic effect exceeding additive effect** | |
|---|---|---|---|---|
| | found (µm) | calc. (µm) | µm | % of calc. effect |
| Extract from fruits of Seronea repens A plus fattty acid alkylesters | 0,88 | 0,58 | ++ 0,30 | + 51,7% |
| Extract from fruits of Seronea repens B plus fatty acid alkylesters | 0,83 | 0,49 | + 0,34 | + 69,4% |
| Pine barke extract A plus fatty acid alkylesters | 0,92 | 0,62 | + 0,30 | + 48,4% |
| Pine bark extract B plus fatty acid alkylesters | 0,87 | 0,46 | + 0,41 | + 89,1% |
| Grape seed extract plus fatty acid alkylesters | 1,07 | 0,78 | + 0,29 | + 37,2% |
| Nettle herb extract plus fatty acid alkylesters | 0,32 | 0,34 | - 0,02 | - 5,9% |
| Extracts from fruits of Seronea repens A plus pine bark extract A | 0,80 | 0,47 | + 0,33 | + 70,2% |
| Extracts from fruits of Seronea repens A plus pine bark extract B | 0,72 | 0,31 | + 0,41 | + 132,2% |
| Extract from fruits of Seronea repens B plus pine bark extract A | 0,62 | 0,38 | +0,24 | +63,2% |
| Extracts from fruits of Seronea repens B plus pine bark extract B | 0,48 | 0,22 | + 0,26 | + 118,2% |
| Extracts from fruits of Seronea repens A plus grape seed extract | 0,88 | 0,63 | + 0,25 | + 39,7% |
| Extract from fruits of Seronea repens B plus grape seed extract | 0,77 | 0,54 | + 0,23 | + 42,6% |

**Tab 3: Increase in hair growth (µm) relative to control in mixtures of three components (found) and calculated as additive effect of the components (calc.)**

| **Components** | | | **Synergistic effect exceeding** | |
|---|---|---|---|---|
| | **Hair growth** | | **additive effect** | |
| | found (µm) | calc. (µm) | µm | % of calc. effect |
| Fatty acid alkylesters plus extract from fruits of Seronea repens A plus pine bark extract A | 1,04 | 0,84 | + 0,20 | +23,8% |
| Fatty acid alkyesters plus exttract from fruits of Seronea repens B plus pine bark extract A | 1,00 | 0,75 | + 0,25 | + 33,3% |
| Fatty acid alkylesters plus exttract from fruits of Seronea repens A plus pine bark extract B | 0,94 | 0,68 | + 0,26 | + 38,2% |
| Fatty acid alkylesters plus exttract from fruits of Seronea repens B plus pine bark extract B | 0,92 | 0,59 | + 0,33 | + 55,9% |
| Fatty acid alkylesters plus exttract from fruits of Seronea repens B plus grape seed extract | 1,14 | 1,0 | + 0,14 | + 14% |

The examples shown in Tab.2 demonstrate the synergistic effect of the combinations. The observed differences of hair growth relative to control are considerably higher than the anticipated values obtained by addition of the growth stimulating effect of their single components. Unexpectedly, the combination of both extracts from *Seronea repens*, known to inhibit the production of dihydrotestosterone (DHT), with another inhibitior of DHT, the fatty acid alkylesters, produced a synergistic effect between 52 and 69 %.

Also surprising was the synergism between the fatty acid alkylesters and plant extracts containing phenol acids and procyanidins, known to act as antioxidants. Both pine bark extracts as well as grape seed extract stimulated hair growth in combination with the fatty acid alkylesters more than could be expected from the stimulating effect of the single components, between 37 and 89 %.

Finally, combinations of both extracs from *Seronea repens* with pine bark or grape seed extracts are more active stimulators of hair growth- by 40 to 132 % - than could be expected from the action of the single components.

The examples in Tab.3 show that the combination of fatty acid alkyl esters with extracts from *Seronea repens* plus pine bark or grape seed extracts was more potent than could be expected from the added contribution of its three single components.

These results of the in vitro experiments with human hair follicles indicate a positive effect of the combination according to the invention on hair growth also in vivo. The substances contained in the mixtures are not metabolised considerably during the very short transfer from the surface of the scalp to the hair roots. Therefore, the hair roots in the scalp are exposed to the same substances, however, in different concentrations compared to the in vitro experiment.

To demonstrate the high efficacy of the combination of fatty acid alkylesters with extracts from *Seronea repens* and procyanidin- and phenolic compounds-containing plant extracts, an investigation was made with human volunteers suffering from alopecia androgenetica.

### Example 2: Controlled study with volunteers

A double-blind, randomized, placebo-controlled study was made with 20 volunteers in each group. The volunteers were diagnosed for alopecia androgenetica.

Study design: The volunteers applied a mixture containing fatty acid alkylesters, an extract from *Seronea repens* and plant extracts containing procyanidins and phenolic acids to the scalp in the morning and in the evening over a period of 6 months.

Volunteers collected fallen-out hairs during each week during the study period, hairs were counted. The number of thick, normal (anagen) hairs was counted by trichoscan at the start of the study within the area affected by hair loss.
Trichoscans were made again after 3, 4, and 6 months.

### Results:

Hair loss was significantly reduced in the second week after application of the mixture and was completely stopped after 3-4 months.

Stimulation of hair growth was detectable after 3 months and highly significant after 6 months.

## Claims

1. Use of a combination comprising alkylesters of fatty acids, of an extract from the fruits of *Seronea repens* and of natural phenolic compounds for the preparation of a composition for the treatment of symptoms of alopecia, preferably alopecia androgenetica.

2. Use according to claim 1 **characterized in that** alkylesters are esterified with fatty acids of chain lengths between C 7 and C 30 , containing between 0 and 2 double bonds and between 1 and 3 hydroxy- or ester groups besides the normal ester group.

3. Use according to claims 1 and 2, further **characterised in that** alkylesters of fatty acids are methyl-,ethyl-,propyl-,isopropyl- or butylesters.

4. Use according to claim 1 **characterized in that** the extract from *Seronea repens* contains the lipophilic constituents of the fruits.

5. Use according to claim 1 **characterized in that** the natural phenolic compounds are selected from natural mixtures containing phenolic acids and flavonoids with phenolic substituents, preferably procyanidins.

6. Use according to claim 1 and 5 **characterized in that** said phenolic compounds are constituents of a plant extract or mixtures of plant extracts.

7. Use according to claim 6, wherein the plant extract or mixture of plant extracts is selected from pine bark extract and/or grape seed extract.

8. Use of a combination according to claim 1 **characterized by** a content of about 1-50% of alkylesters of fatty acids , of about 0.05-20% extract of the fruits from *Seronea repens* and of about 0.01-10% of natural phenolic compounds.

9. Use of a combination according to any of the preceding claims further **characterized by** addition of about 0.1-10% nettle roots extract.

10. Composition comprising alkylesters of fatty acids, an extract from the fruits of *Seronea repens* and natural phenolic compounds.
